# EUROPEAN PATENT APPLICATION

(11) **EP 1 089 205 A2**
(43) Date of publication of application: **04.04.2001**
(21) Application number: 00121603.5
(22) Date of filing: 02.10.2000
(51) Int. Cl.: G06F 17/50

(54) **Method and system for analyzing a catalytic reaction and its utilization**

(30) Priority: 30.09.1999 JP 27955499
(71) Applicant: NEC CORPORATION, Tokyo (JP)
(72) Inventor: Sata, Naoaki, Minato-ku, Tokyo (JP)
(74) Representative: Glawe, Delfs, Moll & Partner

(57) **Abstract**

In a method and system for developing a catalyst, a catalytic reaction is comprehended as a collectivity of elementary reaction processes, which are analyzed by combining an experimental chemistry method, a computational chemistry method, and a computational physics method. The result of the analysis is reflected to the selection of a catalyst construction. Thus, it is possible to realize a systematic and ecological catalyst development having a high degree of generality and simultaneously capable of greatly reducing the time required for the catalyst development.

## Description

### Background of the invention

The present invention relates to a catalytic reaction analyzing method and system, and more specifically to a method and system for analyzing a catalytic reaction by dividing a catalytic reaction into a plurality of elementary reaction processes, and by analyzing each elementary reaction process by means of an optimum method selected from a computational chemistry method, a computational physics method and an experimental chemistry method or a combination of those methods. Furthermore, the present invention relates to a method and system for developing a catalyst by utilizing the catalytic reaction analyzing method and system for selection of controllable parameters

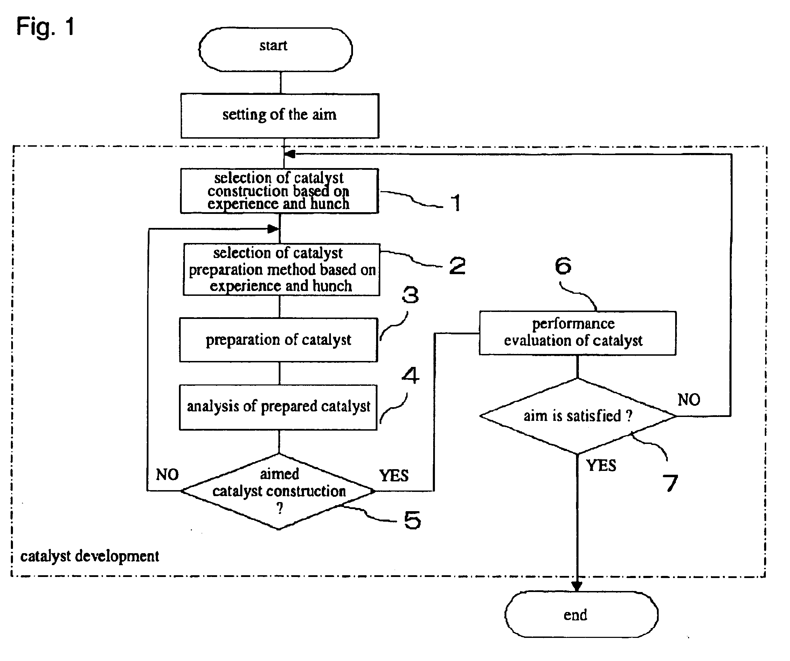

Referring to Fig. 1, there is shown a flow chart illustrating a prior art example of the catalytic reaction developing method. This prior art catalytic reaction developing method is executed: A catalyst construction (active component, and in the case of a heterogeneous catalysis, a catalytic carrier) is selected in accordance with the aim of development (step 1 : selection of catalyst construction), and a method for preparing the catalyst is examined (step 2 : selection of catalyst preparation method). Then, the catalyst is actually prepared (step 3 : preparation of catalyst), and the prepared catalyst is analyzed (step 4 : analysis of prepared catalyst). If it is confirmed that the prepared catalyst has the aimed catalyst construction, it goes to performance evaluation of the catalyst (step 6), and on the other hand, if it is not so confirmed, it returns to the step 2 to re-examine the method for preparing the catalyst and its succeeding steps (step 5 : confirmation as to whether or not the prepared catalyst has the aimed catalyst construction). If the performance evaluation of the catalyst in the step 6 results in that the aim is satisfied, the method ends. On the other hand, if it is evaluated that the aim is not satisfied, it returns to the step 1 so that the method is re-attempted from the selection of catalyst construction.

As regards the selection and re-selection of catalyst construction, no theoretical system exists, and it is an actual practice to perform the selection and re-selection with the experience and the hunch of a developer. For example, a suitable parameter selected from controllable parameters is changed in a suitable direction, and if the performance of the catalyst is resultantly improved, the catalyst is adopted. If not so, the selected parameter is changed in a different direction, or another parameter is selected and changed. Similarly, the selection of the catalyst preparation method is relies on the experience and the hunch. Therefore, a long time is required until the catalyst having the catalyst construction which becomes the object of the evaluation.

In conclusion, the prior art catalytic reaction developing method has the following problems:

A first problem is that huge time and labor are required until a catalyst is developed, and when the aim is achieved (namely, when a desired catalyst is obtained) is inexplicit.

The reason for this is that: As mentioned above, the selection and re-selection of catalyst construction and the selection of the catalyst preparation method, which are a key in the catalyst development, are executed on the basis of the experience and the hunch, which is a uncertain or strongly claptrap means. Particularly, for a completely new aim (reaction), the experience and the hunch cannot be applied, and therefore, the development is in a complete "groping in the dark" condition.

The process for actually preparing (synthesizing) the catalyst and elevating the performance of the prepared catalyst, needs a long time. Generally, the catalyst preparing method is ordinarily different from one catalyst construction to another. A long time needs to be spent for optimizing the catalyst preparing method, for example, for using what catalyst preparing method. If the selection (and re-selection) of catalyst construction is in error, it results in that the process for actually preparing (synthesizing) the catalyst and elevating the performance of the prepared catalyst, which are meaningless from the view of the aimed catalyst, are executed while spending a long time. Therefore, a long time is required until the aimed catalyst can be obtained. Accordingly, it is a matter of course that it is desired to use a mean having as high certainty as possible. Nevertheless, the selection and re-selection of catalyst construction must have been executed by use of the uncertain means such as the experience and the hunch, because (1) there is no means for selecting the catalyst construction unambiguously from a given aim, since an expression mechanism of the function of catalyst is inexplicit and since a causal relation between the catalyst performance and the (selected) catalyst construction is generally inexplicable, (2) there is no general means for add the result obtained from the evaluation of the catalyst performance, as a factor used for deciding the re-selection of catalyst construction, and (3) others. This can be said that, since a method for systematically analyzing the catalyst reaction for a short time has not yet been established, the catalyst reaction cannot be sufficiently analyzed, and therefore, there is nothing than to treat the catalyst having the key of the catalyst reaction, as a black box, with the result that there is nothing than to adopt a trial-and-error developing method as mentioned above.

Efforts are being conducted for shortening the time required for the preparation of the catalyst and the evaluation of the catalyst performance, by introducing a combinatorial chemistry method, as in S. M. Sankan, Nature, 394, 351 (1998). However, the selection and reselection of catalyst construction is still based on the trial-and-error method, and how to change the controllable parameters is still based on the experience and the hunch.

In addition, at present, there are problems attributable to the combinatorial chemistry method itself, for example, a method for preparing a library (preparation or synthesis) and a treating condition therefor are restricted, and a method for analyzing the obtained library is not satisfactory. Accordingly, the introduction of the combinatorial chemistry method does not directly result in a remarkably shortened catalyst developing time.

The circumstances that the huge time is required and it is uncertain when a desired catalyst can be obtained, is a fateful defect. In addition, if the aim is achieved (namely, a desired catalyst can be resultantly obtained), it should be rather considered to be good, but there is possibility that even the aim cannot be resultantly achieved.

A second problem is that a systematic catalyst development cannot be expected in the prior art catalyst developing method. Actually, a material to be treated should have changed into a (final) product by undergoing a plurality of elementary reaction processes under existence of the catalyst. However, in an experimental chemistry method, since only the final product can be ordinarily detected, what can be known is only a final result of the catalytic reaction which is colligated of the elementary reaction processes. A change of a specific controllable parameter should give any influence to a specific elementary reaction process (or a plurality of elementary reaction processes in some cases), but a transition state cannot be directly known by the experimental chemistry method, and an attempt for detecting a reactive intermediate is difficult. As a result, it can be said that it is substantially impossible to directly predict the function of catalyst from the catalyst construction in the frame of the prior art catalyst developing method.

On the other hand, although it is not theoretically supported, there is possibility that the causal relation between the catalyst construction and the function of catalyst can be known to some degree by examining in depth the change in the function of catalyst when respective parameters are changed. In addition, depending upon the kind of the catalytic reaction, it is in some cases that an outline as to what elementary reaction processes the catalytic, reaction undergoes, can be imagined from the result of a series of experiments. However, for this purpose, it is necessary to actually prepare catalysts having different constructions and to analyze the prepared catalysts. This needs many time and labor as mentioned above, and therefore, it is not ordinarily executed.

The problem in which the systematic catalyst development cannot be expected, leads to a third problem in which the catalyst development method has no generality. In the prior art catalyst development method, it is difficult to apply data of development for a catalyst, to development for a completely different catalyst. Accordingly, at present, it is extremely difficult to develop, for a short time, a catalyst in a completely new field (for example, environment catalyst for decomposing a new detrimental substance).

A fourth problem is that a large amount of waste is generated by the catalyst development of the above mentioned trial-and-error type. This must be said to be a significant defect under the present where an environmental quality is becoming important.

In particular, when a detrimental substance (reactant, or product, or catalyst) is handled, a large amount of toxic waste is generated, a further heavy problem may occur. For example, when a catalyst for decomposing 2,3,7,8-tetrachlorodibenzoparadioxin is developed, it is necessary to use the toxic 2,3,7,8-tetrachlorodibenzoparadioxin at each time of evaluating the catalyst performance, and in addition, there is possibility that all of an evaluating machine, labware, consumable goods and the catalyst to be evaluated are contaminated by 2,3,7,8-tetrachlorodibenzoparadioxin. Therefore, those become wastes which need a special control at a discarding time.

### Brief summary of the invention

Accordingly, it is an object of the present invention to provide a catalytic reaction analyzing method which has overcome the above mentioned problems of the prior art catalytic reaction analyzing methods.

Another object of the present invention is to provide a catalytic reaction developing method which utilizes the catalytic reaction analyzing method, and which has overcome the problems of the prior art catalytic reaction developing methods.

According to a first feature of the present invention, the catalytic reaction is comprehended as a collectivity (or group) of elementary reaction processes.

Thus, the catalyst, which was treated as a black box in the prior art, can be grasped as the collectivity of functions which advance the respective elementary reaction processes.

According to a second feature of the present invention, the elementary reaction processes are analyzed and the result of the analysis is fed back to the catalyst development.

In the prior art catalyst development, since the catalyst was treated as a black box, it is difficult to analyze the catalytic reaction, and more difficult to feed back the result of the analysis to the catalyst development. In the present invention, however, by grasping the catalyst as a collectivity of functions which advance the respective elementary reaction processes as mentioned above, it become possible to analyze the catalytic reaction and to feed back the result of the analysis to the catalyst development.

As a result, it is possible to know how the function of catalyst changes because of a change of a controllable parameter, and which of controllable parameters should be changed to obtain a desired function of catalyst. Therefore, a systematic and efficient catalyst development becomes possible.

In addition, catalytic reactions, for example, a catalytic reaction A and a catalytic reaction B, that was considered to be completely different catalytic reactions, may include a common elementary reaction process. In this case, a portion of the analysis result of the catalytic reaction A can be directly deemed as the analysis result of the catalytic reaction B, and can be utilized in a catalyst development for the catalytic reaction B. This can contribute to the efficient catalyst development.

According to a third feature of the present invention, the analysis of the collectivity of elementary reaction processes is carried out by combining an experimental chemistry method, a computational chemistry method and a computational physics method.

In the analysis of the catalytic reaction, it is necessary to accurately know the structure of intermediates and transition states, each composed of a catalyst and a reactive chemical species bonding with the catalyst, and stability of the intermediates and the and transition states. As mentioned above, however, it is impossible to directly know the transition state by use of only the experimental chemistry method, and it is difficult to detect the intermediates by use of only the experimental chemistry method. Accordingly, a catalytic reaction analysis satisfactory for the catalyst development cannot be realized by directly utilizing only the experimental chemistry method.

According to the computational chemistry method, it is possible to know the intermediates and the and transition states by computation. In a pharmaceutical field and others, actually, a molecular design is conducted by utilizing the computational chemistry method. However, since a solid substance cannot be handled in the computational chemistry method, it was difficult to analyze a catalytic reaction by use of only the computational chemistry method, in a solid catalyst such as a solid phase-gas phase heterogeneous catalysis in which a physical property of a solid matter is significant, and in a homogeneous catalysis in which large clusters engage in the catalytic reaction.

However, the computational physics method can handle the solid substance, and is already applied in developing various physical materials. However, since the computational physics method cannot handle the chemical reaction, it is difficult to analyze the catalytic reaction by use of only the computational physics method.

As mentioned above, each of the experimental chemistry method, the computational chemistry method, and the computational physics method has advantages and disadvantages, and therefore, the catalytic reaction cannot be directly analyzed by use of only one of the experimental chemistry method, the computational chemistry method, and the computational physics method.

On the other hand, in the present invention, the catalytic reaction is comprehended as a collectivity of elementary reaction processes. Each of the elementary reaction processes is analyzed by selecting an optimum one from the group consisting of the experimental chemistry method, the computational chemistry method, and the computational physics method, a combination of two selected from the experimental chemistry method, the computational chemistry method, and the computational physics method, and a combination of the experimental chemistry method, the computational chemistry method, and the computational physics method, so that the catalytic reaction analysis becomes extremely efficient and effective in comparison with the case that the catalytic reaction analysis is conducted by applying any one of the experimental chemistry method, the computational chemistry method, and the computational physics method, to an overall catalytic reaction.

According to a fourth feature of the present invention, a preliminary catalyst performance evaluation is conducted by the above mentioned catalyst analysis method, prior to an actual preparation of the catalyst.

In the present invention, it is sufficient if it is possible to prepare only catalysts required when the preliminary catalyst performance evaluation is conducted by the experimental chemistry method, and a catalyst presumed to meet the aim in accordance with the preliminary catalyst performance evaluation. Therefore, it is possible to greatly reduce the time and the labor which are required for the step for selecting the catalyst preparation method and its succeeding steps.

In addition, the possibility of greatly reducing the time and the labor which are required for the step for selecting the catalyst preparation method and its succeeding steps, leads to possibility of greatly reducing the waste generated in the process of the catalyst development. Therefore, an ecological catalyst development can he realized in accordance with the present invention. For example, even in the case of handling a toxic material as in developing a catalyst for decomposing 2,3,78-tetrachlorodibenzoparadioxin, it is possible to reduce a risky experiment handling such a toxic material and simultaneously to minimize generation of toxic materials.

Here, in this specification, the following terms should be understood to have the following meanings:

The "catalyst" is defined to be a substance that changes the kinetics of a chemical reaction by adding the substance into a system in which the chemical reaction can advance thermodynamically. Ordinary, the condition of the catalyst includes a requisition that the substance does not change before and after the reaction. In this specification, however, even if the substance does not satisfy this requisition, the substance is deemed to be the catalyst. For example, a substance such as a chemical adsorbent, which is not ordinarily deemed as the catalyst, is included in the catalyst in this specification.

The "catalytic reaction" is defined to be a chemical reaction that can advance in a system including the catalyst, by action of the catalyst. In this specification, the catalytic reaction should be understood to include not only a homogeneous catalytic reaction and a heterogeneous catalytic reaction which are an ordinary catalytic reaction, but also deactivation of catalysis, and a chemical adsorption by a chemical adsorbent

The "catalytic reaction process" is what expresses an advancing route of the above mentioned catalytic reaction.

The "elementary reaction" is a reaction that the change of a chemical bond is expressed by one microscopic process in a system including at least one chemical species such as a molecule, an atom, a solid substance, a radical, an ion, etc. Here, the "bond" should be understood to include not only a covalent bond and an ionic bond, but also all other bonds for bonding between chemical species including a molecule, an atom, a solid substance, a radical, and an ion, by action of a intermolecular force or another force, for example, a bond given by a weak intermolecular force such as a van der Waals force acting between chemical species. Accordingly, in this specification, the elementary reaction includes a physical absorbing phenomenon at a solid substance surface.

The "elementary reaction process" is what expresses an advancing route of the above mentioned elementary reaction. As mentioned above, in this specification, since the elementary reaction includes the physical absorbing phenomenon attributable to the weak intermolecular force, the elementary reaction process should be considered to be include a phenomenon such as adsorption to the catalyst and detachment from the catalyst, which are ordinarily considered to be a physical phenomenon.

The "controllable parameter" is a parameter that can be selected within any or a specific range in an aimed catalytic reaction process. In an ordinary catalytic reaction, a reaction condition such as a treating temperature and a treating flow rate, a catalyst construction (including a catalyst composition), and a carrier, are this controllable parameter. In this case, if a starting material of the catalytic reaction can be selected from different kinds of compound, the starting material becomes the controllable parameter. To the contrary, in a catalytic reaction for converting a specified starting material, for example, a toxic waste, into a product having no toxic property, by action of a catalyst, the starting material is not the controllable parameter, but the product becomes the controllable parameter.

The "catalytic reaction analysis" is an analysis for knowing a causal relation between the change of the controllable parameter and the function of catalyst.

The "intermediate" is a chemical species that is generated in the way of the reaction route starting from the starting material and to reaching the product in the catalytic reaction. In this specification, the intermediate includes a intermediate have an extremely short lifetime which cannot be captured in an ordinary chemical analysis.

The "transition state" is a condition in which a free energy of the system is maximum in the reaction route.

The "elementary reaction process analysis" is to know the change, in the elementary reaction process, of the physical property of respective chemical species (molecule, atom, solid substance, radical, ion, etc.) engaging in the reaction, and the energy state of the system, at a starting point and at an ending point of the elementary reaction process, in a intermediate, and in a transition state.

The "computational physics method" is an analyzing method mainly for computing an interaction between an electron and a nucleus on the basis of the quantum mechanics, to simulate the physical property having a directly engaging electronic structure.

The "computational chemistry method" is an analyzing method mainly for computing an interaction between chemical species (molecule, atom, solid substance, radical, ion, etc.) on the basis of the quantum mechanics, to simulate an interaction and a reaction between chemical species.

The "experimental chemistry method" is an analyzing method based on an experiment such as a chemical experiment and a chemical analysis, accompanied with a chemical change, a physical property evaluation using an instrumental analysis, and the result thereof.

The above and other objects, features and advantages of the present invention will be apparent from the following description of preferred embodiments of the invention with reference to the accompanying drawings.

### Brief description of the drawings

Fig. 1 is a flow chart illustrating the prior art catalyst developing method;
Fig. 2 is a flow chart illustrating one embodiment of the catalyst developing method in accordance with the present invention; and
Fig. 3 is a diagrammatic block diagram illustrating one embodiment of the catalyst developing system in accordance with the present invention.

### Detailed description of the invention

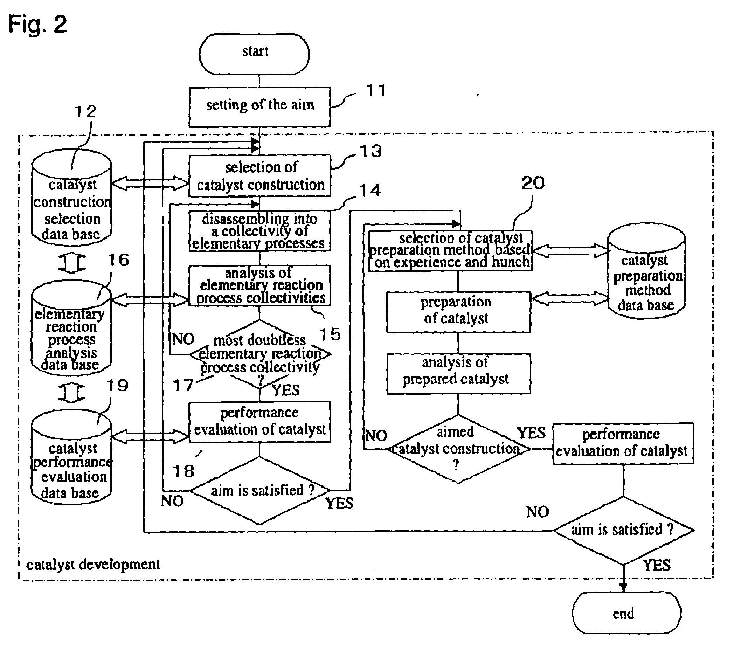

Referring to Fig. 2, there is shown a flow chart illustrating one embodiment of the catalyst developing method in accordance with the present invention.

First, a catalytic reaction which is an aim to be conducted with a catalyst is set (step 11 : setting of the aim). Then, a catalyst construction is selected on the basis of data in a catalyst construction selection data base 12 (step 13 : selection of catalyst construction).

Thereafter, the catalytic reaction is disassembled into a collectivity of elementary reaction processes (step 14 : disassembly into a collectivity of elementary reaction processes). Generally, a plurality of elementary reaction process collectivities can be considered for one catalytic reaction. Each elementary reaction process collectivity is analyzed by applying a suitable one selected from the experimental chemistry method, the computational chemistry method and the computational physics method to respective elementary reaction processes (step 15 : analysis of the collectivities of elementary reaction processes). Here, as mentioned above, it is not necessary to apply the same analyzing method for all the elementary reaction processes. The result of the analysis of the respective elementary reaction process collectivities is compared with the result of the analysis by the experimental chemistry method for the overall catalytic reaction, or alternatively with the result of another analysis already stored in an elementary reaction process analysis data base 16, and then, the result of computation having the highest degree of coincidence is regarded as the most doubtless elementary reaction process collectivity. Here, the analyzing method used in this process is regarded as an analyzing method which is estimated to be the most efficient analyzing method for analyzing each elementary reaction process (step 17 : selection of the elementary reaction process collectivity and the analyzing method).

For the selection of the most doubtless elementary reaction process collectivity and for the assignment of the most efficient analyzing method, an evolutionary computation for example, a genetic algorithm can be applied.

Just after the start of an operation of this embodiment, the catalyst construction selection data base 12 stores the experience and the hunch in prior catalyst developing methods. However, with the operation of this embodiment, more effective information is additionally accumulated in the catalyst construction selection data base 12.

The result of analysis is accumulated in the elementary reaction process analysis data base 16, by sorting the result of analysis by elementary reaction process. Therefore, the accumulated result of analysis can be immediately utilized at the time of analyzing another reaction process, or at the time of verifying another analysis result.

It cannot be necessarily said that the most doubtless elementary reaction process collectivity obtained from the above analysis gives true elementary reaction processes. In addition, it cannot be necessarily said that the most efficient analyzing method is a truly most efficient analyzing method. However, the degree of doubtlessness can be elevated by repeating the above mentioned process in which the result of computation for the respective elementary reaction process collectivities is compared with the result by the experimental chemistry method for the overall catalytic reaction.(the verification by the experimental chemistry method), or alternatively, with the result of another analysis stored in the elementary reaction process analysis data base 16, so that the result of computation having the highest degree of coincidence is regarded as a most doubtless elementary reaction process collectivity.

The computational chemistry method, the computational physics method and the experimental chemistry method used in the present invention are not limited, and conventionally used methods can be used without modification. As regards the experimental chemistry method, a combinatorial chemistry method which is becoming recently used in a pharmaceutical field, can be used in addition to a surface analyzing method and a catalyst evaluating method which are used traditionally. For example, since most of phenomena occurring on a catalyst surface advances on the order of femtoseconds, it is now difficult to directly capture the phenomenon, but a resultantly generated phenomenon, for example, an oscillating excited condition of the product is maintained for a length of time longer than femtoseconds, and therefore, can be detected in some cases. D. J. Balds and S. L. Bemasek, J. Chem. Phys., 109. 746 (1998) succeeded in estimating a reaction mechanism by observing this oscillating excited condition. This method can know only the elementary reaction processes constituting a portion of a limited catalytic reaction, but can be applied in the present invention as a matter of course if possible.

In the present invention, no matter what analyzing method is used, the present invention can finally reach an optimum analyzing method by a learning of the system based on the verification of the above mentioned analysis result. However, a manner of preferentially using a method which is experientially considered to be effective for an efficient analysis, would be effective in shortening the analyzing time, and therefore, is recommendable.

For example, whether or not the method is optimum is decided on the basis of the feature of respective elementary reaction processes. In a system in which information concerning the physical property is important, the computational physics method is preferentially used. In a system in which information concerning the reaction is important, the computational chemistry method is used.

Consider as an example a catalytic reaction including (1) adsorption to a catalyst surface of a substance to be treated, (2) shuffle of the bond on the catalyst surface, and (3) detachment of the produce from the catalyst surface. In the processes (1) and (3), it is important to analyze the physical property on the catalyst surface, in addition to analyze the elementary reaction processes for these processes. In the process (2), it is important to accurately know the structure of intermediates and transition states, which are composed of a reactive chemical species bonding with the catalyst, and stability of the intermediates and the and transition states. In this example, an efficient reaction analysis can be conducted by using the computational physics method in analyzing the physical property on the catalyst surface in the process (1), and by using the computational chemistry method in analyzing a reaction situation in the processes (1) and (2).

Dependently upon the kind of the catalytic reaction, the analysis can be completed by only one or two of the above mentioned three methods. However, it is preferred that the three methods can be used always to comply with any reaction.

On the basis of the result of the analysis, a catalyst performance evaluation is conducted (step 18). Specifically, the catalyst performance evaluation (step 18) is conducted on the basis of the data in a catalyst performance evaluation data base 19. The catalyst construction selection data base 12, the elementary reaction process analysis data base 16, and the catalyst performance evaluation data base 19 can share data. Alternatively, it may be in some cases that just after the start of the operation in the system, no effective data is stored in the catalyst performance evaluation data base 19. In this case, this step is skipped so that it goes into the selection of the catalyst preparation method (step 20).

The catalyst preparation method selection 20 and its succeeding steps are apparently similar to the prior art method explained with reference to Fig. 1, and therefore, further explanation will be omitted. However, since the catalyst previously restricted to possible catalyst candidates before it goes into the catalyst preparation method selection step, the number of catalyst candidates to be analyzed is greatly reduced in comparison with the prior art, and the time and the labor can be greatly reduced.

Here, the computational chemistry method and the experimental chemistry method are not necessarily required to be on the same computer as that having a computer program product for the catalytic reaction analysis in accordance with the present invention. This is also true in connection with the data bases. The following embodiment is a system in which it is possible to contact with the computational chemistry method and the experimental chemistry method through a network.

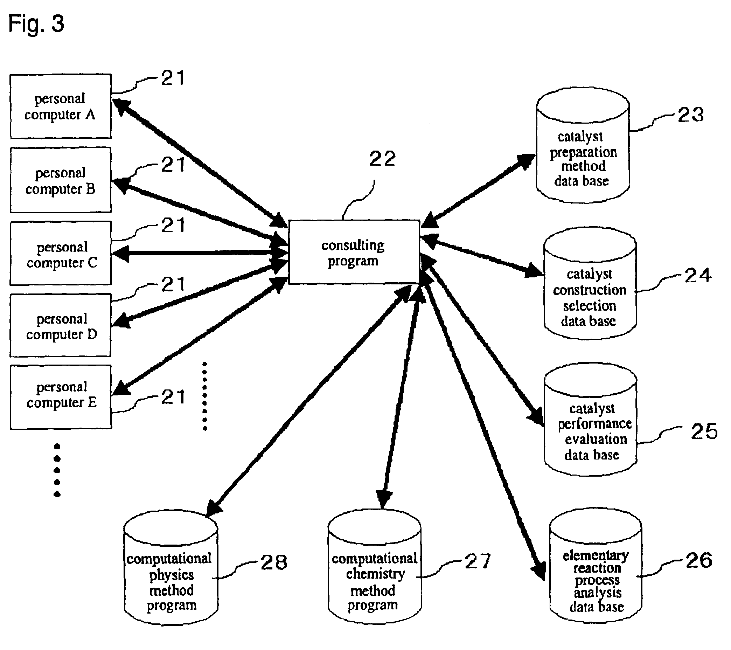

Referring to Fig. 3, there is shown a diagrammatic block diagram illustrating one embodiment of the catalyst developing system in accordance with the present invention, so configured that the computer program product for the catalytic reaction analysis in accordance with the present invention, excluding the data bases, (called a "consulting program" hereinafter), and the base bases are distributed on a network. A computer 22 including the consulting program is connected through a network to data bases 23, 24, 25 and 26, analyzing programs 27 and 28 for the computational chemistry method and the computational physics method, and personal computers 21 for catalyst developers.

In this embodiment, the catalyst developers can connect their personal computer through the network to the computer 22 including the consulting program, the catalyst developers can conduct the catalyst development and the catalytic reaction analysis anywhere. In addition, since each catalyst developer can share the data and the analysis result of other catalyst developers, each catalyst developer can efficiently develop the catalyst.

As mentioned above, according to the present invention, it is possible to realize a systematic and ecological catalyst development having a high degree of generality, and at the same time to greatly reduce the time required for the catalyst development.

Finally, the content of Japanese Patent Application No. 279554/1999 filed on September 30, 1999 is incorporated by reference in its entirety into this application.

The invention has thus been shown and described with reference to the specific embodiments. However, it should be noted that the present invention is in no way limited to the details of the illustrated structures but changes and modifications may be made within the scope of the appended claims.

## Claims

1. A system for selecting, when a catalytic reaction process is expressed as a collectivity of elementary reaction processes, the most doubtless elementary reaction process collectivity from a plurality of expressible elementary reaction process collectivities,
the "catalytic reaction process" being what expresses an advancing route of a catalytic reaction, the "catalytic reaction" being defined to be a chemical reaction that can advance in a system including the catalyst, by action of the catalyst, the "catalyst" being defined to be a substance that changes the velocity of a chemical reaction by adding the substance into a system in which the chemical reaction can advance thermodynamically,
the "elementary reaction process collectivity" being a plurality of continuous elementary reaction processes or a collectivity of elementary reaction processes, the elementary reaction process" being what expresses an advancing route of the elementary reaction, the "elementary reaction" being a reaction that the change of a bond is expressed by one microscopic process in a system including at least one chemical species including a molecule, an atom, a solid substance, a radical, and an ion, the "bond" being defined to include a covalent bond, an ionic bond and all other bonds for bonding between chemical species including a molecule, an atom, a solid substance, a radical, and an ion, by action of a intermolecular force or another force,
wherein the system executes:
the step of analyzing each expressible elementary reaction process collectivity by applying a suitable one selected from the experimental chemistry method, the computational chemistry method and the computational physics method to respective elementary reaction processes of each elementary reaction process collectivity, where the "experimental chemistry method" is an analyzing method based on an experiment such as a chemical experiment and a chemical analysis, accompanied with a chemical change, a physical property evaluation using an instrumental analysis, and the result thereof, the "computational chemistry method" is an analyzing method mainly for computing an interaction between chemical species (molecule, atom, solid substance, radical, ion, etc.) on the basis of the quantum mechanics, to simulate an interaction and a reaction between chemical species, the "computational physics method" is an analyzing method mainly for computing an interaction between an electron and a nucleus on the basis of the quantum mechanics, to simulate the physical property having a directly engaging electronic structure, and the "analysis" is to know the change, in the elementary reaction process, of the physical property of respective chemical species (molecule, atom, solid substance, radical, ion, etc.) engaging in the reaction, and the energy state of the system, at a starting point and at an ending point of the elementary reaction process, in a reactive intermediate, and in a transition state,
the step of regarding a combination of the result of the analysis of the respective elementary reaction processes of each elementary reaction process collectivity as the result of the analysis of the elementary reaction process collectivity;
the step of comparing the result of the analysis of each elementary reaction process collectivity with the result of the analysis by the experimental chemistry method for the overall catalytic reaction;
the step of selecting an elementary reaction process collectivity having the highest degree of coincidence, as the most doubtless elementary reaction process collectivity.

2. A system for selecting an analyzing method which is estimated to be the most efficient, when an analysis is conducted for an elementary reaction process included a catalytic reaction process,
the "catalytic reaction process" being what expresses an advancing route of a catalytic reaction, the "catalytic reaction" being defined to be a chemical reaction that can advance in a system including the catalyst, by action of the catalyst, the "catalyst" being defined to be a substance that changes the velocity of a chemical reaction by adding the substance into a system in which the chemical reaction can advance thermodynamically, the "analysis" being to know the change, in the elementary reaction process, of the physical property of respective chemical species (molecule, atom, solid substance, radical, ion, etc.) engaging in the reaction, and the energy state of the system, at a starting point and at an ending point of the elementary reaction process, in a reactive intermediate, and in a transition state,
the elementary reaction process" being what expresses an advancing route of the elementary reaction, the "elementary reaction" being a reaction that the change of a bond is expressed by one microscopic process in a system including at least one chemical species including a molecule, an atom, a solid substance, a radical, and an ion, the "bond" being defined to include a covalent bond, an ionic bond and all other bonds for bonding between chemical species including a molecule, an atom, a solid substance, a radical, and an ion, by action of a intermolecular force or another force,
wherein the system executes:
the step of analyzing each expressible elementary reaction process collectivity by applying a suitable one selected from the experimental chemistry method, the computational chemistry method and the computational physics method to respective elementary reaction processes of each elementary reaction process collectivity, where the "elementary reaction process collectivity" is a plurality of continuous elementary reaction processes or a collectivity of elementary reaction processes, the "experimental chemistry method" is an analyzing method based on an experiment such as a chemical experiment and a chemical analysis, accompanied with a chemical change, a physical property evaluation using an instrumental analysis, and the result thereof, the "computational chemistry method" is an analyzing method mainly for computing an interaction between chemical species (molecule, atom, solid substance, radical, ion, etc.) on the basis of the quantum mechanics, to simulate an interaction and a reaction between chemical species, and the "computational physics method" is an analyzing method mainly for computing an interaction between an electron and a nucleus on the basis of the quantum mechanics, to simulate the physical property having a directly engaging electronic structure,
the step of regarding a combination of the result of the analysis of the respective elementary reaction processes of each elementary reaction process collectivity as the result of the analysis obtained when the respective analyzing methods are assigned to the respective elementary reaction process collectivity;
the step of comparing the result of the analysis of respective elementary reaction process collectivity when the respective analyzing methods are assigned, with the result of the analysis by the experimental chemistry method for the overall catalytic reaction;
the step of selecting an analyzing method assignment giving the result of the analysis having the highest degree of coincidence, as a analyzing method assignment which is estimated to have the highest efficiency;
the step of selecting respective analyzing methods assigned to respective elementary reaction processes in the analyzing method assignment giving the result of the analysis having the highest degree of coincidence, as the analyzing method which is estimated to be the most efficient for the respective elementary reaction processes.

3. A system claimed in Claim 2, wherein when a catalytic reaction process is expressed as a collectivity of elementary reaction processes, the most doubtless elementary reaction process collectivity is selected from a plurality of expressible elementary reaction process collectivities, and when an analysis is conducted for an elementary reaction process included a catalytic reaction process, an analyzing method which is estimated to be the most efficient, is selected,
wherein after the comparison with the result of the analysis by the experimental chemistry method for the overall catalytic reaction, an elementary reaction process collectivity having the highest degree of coincidence is selected as the most doubtless elementary reaction process collectivity, and respective analyzing methods assigned to respective elementary reaction processes of the most doubtless elementary reaction process collectivity, are selected as the analyzing method which is estimated to be the most efficient for the respective elementary reaction processes.

4. A computer program product used in a system for selecting, when a catalytic reaction process is expressed as a collectivity of elementary reaction processes, the most doubtless elementary reaction process collectivity from a plurality of expressible elementary reaction process collectivities,
the "catalytic reaction process" being what expresses an advancing route of a catalytic reaction, the "catalytic reaction" being defined to be a chemical reaction that can advance in a system including the catalyst, by action of the catalyst, the "catalyst" being defined to be a substance that changes the velocity of a chemical reaction by adding the substance into a system in which the chemical reaction can advance thermodynamically,
the "elementary reaction process collectivity" being a plurality of continuous elementary reaction processes or a collectivity of elementary reaction processes, the elementary reaction process" being what expresses an advancing route of the elementary reaction, the "elementary reaction" being a reaction that the change of a bond is expressed by one microscopic process in a system including at least one chemical species including a molecule, an atom, a solid substance, a radical, and an ion, the "bond" being defined to include a covalent bond, an ionic bond and all other bonds for bonding between chemical species including a molecule, an atom, a solid substance, a radical, and an ion, by action of a intermolecular force or another force,
wherein the computer program product includes a recording medium which can be used in a computer, said recording medium recording a computer program including:
the step of analyzing each expressible elementary reaction process collectivity by applying a suitable one selected from the experimental chemistry method, the computational chemistry method and the computational physics method to respective elementary reaction processes of each elementary reaction process collectivity;
the step of regarding a combination of the result of the analysis of the respective elementary reaction processes of each elementary reaction process collectivity as the result of the analysis of the elementary reaction process collectivity;
the step of comparing the result of the analysis of each elementary reaction process collectivity with the result of the analysis by the experimental chemistry method for the overall catalytic reaction;
the step of selecting an elementary reaction process collectivity having the highest degree of coincidence, as the most doubtless elementary reaction process collectivity.
where the "experimental chemistry method" is an analyzing method based on an experiment such as a chemical experiment and a chemical analysis, accompanied with a chemical change, a physical property evaluation using an instrumental analysis, and the result thereof, the "computational chemistry method" is an analyzing method mainly for computing an interaction between chemical species (molecule, atom, solid substance, radical, ion, etc.) on the basis of the quantum mechanics, to simulate an interaction and a reaction between chemical species, the "computational physics method" is an analyzing method mainly for computing an interaction between an electron and a nucleus on the basis of the quantum mechanics, to simulate the physical property having a directly engaging electronic structure, and the "analysis" is to know the change, in the elementary reaction process, of the physical property of respective chemical species (molecule, atom, solid substance, radical, ion, etc.) engaging in the reaction, and the energy state of the system, at a starting point and at an ending point of the elementary reaction process, in a reactive intermediate, and in a transition state,

5. A computer program product used in a system for selecting an analyzing method which is estimated to be the most efficient, when an analysis is conducted for an elementary reaction process included a catalytic reaction process,
the "catalytic reaction process" being what expresses an advancing route of a catalytic reaction, the "catalytic reaction" being defined to be a chemical reaction that can advance in a system including the catalyst, by action of the catalyst, the "catalyst" being defined to be a substance that changes the velocity of a chemical reaction by adding the substance into a system in which the chemical reaction can advance thermodynamically, the "analysis" being to know the change, in the elementary reaction process, of the physical property of respective chemical species (molecule, atom, solid substance, radical, ion, etc.) engaging in the reaction, and the energy state of the system, at a starting point and at an ending point of the elementary reaction process, in a reactive intermediate, and in a transition state,
the elementary reaction process" being what expresses an advancing route of the elementary reaction, the "elementary reaction" being a reaction that the change of a bond is expressed by one microscopic process in a system including at least one chemical species including a molecule, an atom, a solid substance, a radical, and an ion, the "bond being defined to include a covalent bond, an ionic bond and all other bonds for bonding between chemical species including a molecule, an atom, a solid substance, a radical, and an ion, by action of a intermolecular force or another force.
wherein the computer program product includes a recording medium which can be used in a computer, said recording medium recording a computer program including:
the step of analyzing each expressible elementary reaction process collectivity by applying a suitable one selected from the experimental chemistry method, the computational chemistry method and the computational physics method to respective elementary reaction processes of each elementary reaction process collectivity;
the step of regarding a combination of the result of the analysis of the respective elementary reaction processes of each elementary reaction process collectivity as the result of the analysis obtained when the respective analyzing methods are assigned to the respective elementary reaction process collectivity;
the step of comparing the result of the analysis of respective elementary reaction process collectivity when the respective analyzing methods are assigned, with the result of the analysis by the experimental chemistry method for the overall catalytic reaction;
the step of selecting an analyzing method assignment giving the result of the analysis having the highest degree of coincidence, as a analyzing method assignment which is estimated to have the highest efficiency;
the step of selecting respective analyzing methods assigned to respective elementary reaction processes in the analyzing method assignment giving the result of the analysis having the highest degree of coincidence, as the analyzing method which is estimated to be the most efficient for the respective elementary reaction processes,
where the "elementary reaction process collectivity" is a plurality of continuous elementary reaction processes or a collectivity of elementary reaction processes, the "experimental chemistry method" is an analyzing method based on an experiment such as a chemical experiment and a chemical analysis, accompanied with a chemical change, a physical property evaluation using an instrumental analysis, and the result thereof, the "computational chemistry method" is an analyzing method mainly for computing an interaction between chemical species (molecule, atom, solid substance, radical, ion, etc.) on the basis of the quantum mechanics, to simulate an interaction and a reaction between chemical species, and the "computational physics method" is an analyzing method mainly for computing an interaction between an electron and a nucleus on the basis of the quantum mechanics, to simulate the physical property having a directly engaging electronic structure,

6. A computer program product claimed in Claim 5, wherein said computer program is so configured to select, when a catalytic reaction process is expressed as a collectivity of elementary reaction processes, the most doubtless elementary reaction process collectivity is selected from a plurality of expressible elementary reaction process collectivities, and to select an analyzing method which is estimated to be the most efficient, when an analysis is conducted for an elementary reaction process included a catalytic reaction process,
wherein said computer program includes, after the comparison with the result of the analysis by the experimental chemistry method for the overall catalytic reaction, the step of selecting an elementary reaction process collectivity giving the result of the analysis having the highest degree of coincidence as the most doubtless elementary reaction process collectivity, and respective analyzing methods assigned to respective elementary reaction processes of the most doubtless elementary reaction process collectivity, are selected as the analyzing method which is estimated to be the most efficient for the respective elementary reaction processes.

7. A system for analyzing a catalytic reaction by expressing a catalytic reaction process as a collectivity of elementary reaction processes,
the "catalytic reaction process" being what expresses an advancing route of a catalytic reaction, the "catalytic reaction" being defined to be a chemical reaction that can advance in a system including the catalyst, by action of the catalyst, the "catalyst" being defined to be a substance that changes the velocity of a chemical reaction by adding the substance into a system in which the chemical reaction can advance thermodynamically,
the "elementary reaction process collectivity" being a plurality of continuous elementary reaction processes or a collectivity of elementary reaction processes, the elementary reaction process" being what expresses an advancing route of the elementary reaction, the "elementary reaction" being a reaction that the change of a bond is expressed by one microscopic process in a system including at least one chemical species including a molecule, an atom, a solid substance, a radical, and an ion, the "bond" being defined to include a covalent bond, an ionic bond and all other bonds for bonding between chemical species including a molecule, an atom, a solid substance, a radical, and an ion, by action of a intermolecular force or another force,
wherein the system executes:
the step of analyzing each expressible elementary reaction process collectivity by applying a suitable one selected from the experimental chemistry method, the computational chemistry method and the computational physics method to respective elementary reaction processes of each elementary reaction process collectivity, where the "experimental chemistry method" is an analyzing method based on an experiment such as a chemical experiment and a chemical analysis, accompanied with a chemical change, a physical property evaluation using an instrumental analysis, and the result thereof, the "computational chemistry method" is an analyzing method mainly for computing an interaction between chemical species (molecule, atom, solid substance, radical, ion, etc.) on the basis of the quantum mechanics, to simulate an interaction and a reaction between chemical species, the "computational physics method" is an analyzing method mainly for computing an interaction between an electron and a nucleus on the basis of the quantum mechanics, to simulate the physical property having a directly engaging electronic structure, and the "analysis" is to know the change, in the elementary reaction process, of the physical property of respective chemical species (molecule, atom, solid substance, radical, ion, etc.) engaging in the reaction, and the energy state of the system, at a starting point and at an ending point of the elementary reaction process, in a reactive intermediate, and in a transition state,
the step of regarding a combination of the result of the analysis of the respective elementary reaction processes of each elementary reaction process collectivity as the result of the analysis of the elementary reaction process collectivity;
the step of comparing the result of the analysis of each elementary reaction process collectivity with the result of the analysis by the experimental chemistry method for the overall catalytic reaction;
the step of selecting an elementary reaction process collectivity having the highest degree of coincidence, as the most doubtless elementary reaction process collectivity, to regard the result of the analysis of the selected elementary reaction process collectivity as the result of the analysis of the catalytic reaction.

8. A system claimed in Claim 7, wherein the system executes:
the step of regarding a combination of the result of the analysis of the respective elementary reaction processes of each elementary reaction process collectivity as the result of the analysis obtained when the respective analyzing methods are assigned to the respective elementary reaction process collectivity;
the step of comparing the result of the analysis of respective elementary reaction process collectivity when the respective analyzing methods are assigned, with the result of the analysis by the experimental chemistry method for the overall catalytic reaction;
the step of selecting an analyzing method assignment giving the result of the analysis having the highest degree of coincidence, as a analyzing method assignment which is estimated to have the highest efficiency;
the step of selecting an analyzing method assignment giving the result of the analysis having the highest degree of coincidence, as the analyzing method assignment which is estimated to be the most efficient, to regard the result of the analysis in the selected analyzing method assignment as the result of the analysis of the catalytic reaction.

9. A system claimed in Claim 8, wherein after the comparison with the result of the analysis by the experimental chemistry method for the overall catalytic reaction, all elementary reaction process collectivity having the highest degree of coincidence is selected as the most doubtless elementary reaction process collectivity, and the respective analyzing methods assigned to respective elementary reaction processes of the selected elementary reaction process collectivity, are selected by regarding as the analyzing method which is estimated to be the most efficient for the respective elementary reaction process, to regard the result of the analysis in the selected analyzing method as the result of the analysis of the catalytic reaction.

10. A computer program product used in a system for analyzing a catalytic reaction, the computer program product including a recording medium which can be used in a computer, where a catalytic reaction process being expressed as a collectivity of elementary reaction processes,
the "catalytic reaction process" being what expresses an advancing route of a catalytic reaction, the "catalytic reaction" being defined to be a chemical reaction that can advance in a system including the catalyst, by action of the catalyst, the "catalyst" being defined to be a substance that changes the velocity of a chemical reaction by adding the substance into a system in which the chemical reaction can advance thermodynamically,
the "elementary reaction process collectivity" being a plurality of continuous elementary reaction processes or a collectivity of elementary reaction processes, the elementary reaction process" being what expresses an advancing route of the elementary reaction, the "elementary reaction" being a reaction that the change of a bond is expressed by one microscopic process in a system including at least one chemical species including a molecule, an atom, a solid substance, a radical, and an ion, the "bond" being defined to include a covalent bond, an ionic bond and all other bonds for bonding between chemical species including a molecule, an atom, a solid substance, a radical, and an ion, by action of a intermolecular force or another force,
said computer program including:
the step of analyzing each expressible elementary reaction process collectivity by applying a suitable one selected from the experimental chemistry method, the computational chemistry method and the computational physics method to respective elementary reaction processes of each elementary reaction process collectivity, where the "experimental chemistry method" is an analyzing method based on an experiment such as a chemical experiment and a chemical analysis, accompanied with a chemical change, a physical property evaluation using an instrumental analysis, and the result thereof, the "computational chemistry method" is an analyzing method mainly for computing an interaction between chemical species (molecule, atom, solid substance, radical, ion, etc.) on the basis of the quantum mechanics, to simulate an interaction and a reaction between chemical species, the "computational physics method" is an analyzing method mainly for computing an interaction between an electron and a nucleus on the basis of the quantum mechanics, to simulate the physical property having a directly engaging electronic structure, and the "analysis" is to know the change, in the elementary reaction process, of the physical property of respective chemical species (molecule, atom, solid substance, radical, ion, etc.) engaging in the reaction, and the energy state of the system, at a starting point and at an ending point of the elementary reaction process, in a reactive intermediate, and in a transition state;
the step of regarding a combination of the result of the analysis of the respective elementary reaction processes of each elementary reaction process collectivity as the result of the analysis of the elementary reaction process collectivity;
the step of comparing the result of the analysis of each elementary reaction process collectivity with the result of the analysis by the experimental chemistry method for the overall catalytic reaction;
the step of selecting an elementary reaction process collectivity having the highest degree of coincidence, as the most doubtless elementary reaction process collectivity, to regard the result of the analysis of the selected elementary reaction process collectivity as the result of the analysis of the catalytic reaction.

11. A computer program product claimed in Claim 10, wherein said computer program includes:
the step of regarding a combination of the result of the analysis of the respective elementary reaction processes of each elementary reaction process collectivity as the result of the analysis obtained when the respective analyzing methods are assigned to the respective elementary reaction process collectivity;
the step of comparing the result of the analysis of respective elementary reaction process collectivity when the respective analyzing methods are assigned, with the result of the analysis by the experimental chemistry method for the overall catalytic reaction;
the step of selecting an analyzing method assignment giving the result of the analysis having the highest degree of coincidence, as a analyzing method assignment which is estimated to have the highest efficiency, to regard the result of the analysis in the selected analyzing method assignment as the result of the analysis of the catalytic reaction.

12. A computer program product claimed in Claim 10, wherein said computer program includes, after the comparison with the result of the analysis by the experimental chemistry method for the overall catalytic reaction, the step of selecting an elementary reaction process collectivity giving the result of the analysis having the highest degree of coincidence as the most doubtless elementary reaction process collectivity, and the step of selecting a respective analyzing method assigned to respective elementary reaction process of the most doubtless elementary reaction process collectivity, as the analyzing method which is estimated to be the most efficient for the same elementary reaction process
to regard the result of the analysis of the selected elementary reaction process collectivity as the result of the analysis of the catalytic reaction.

13. A system for developing a catalyst, wherein a catalytic reaction process is expressed as a collectivity of elementary reaction processes,
the "catalyst" being defined to be a substance that changes the velocity of a chemical reaction by adding the substance into a system in which the chemical reaction can advance thermodynamically, the "catalytic reaction process" being what expresses an advancing route of a catalytic reaction, the "catalytic reaction" being defined to be a chemical reaction that can advance in a system including the catalyst, by action of the catalyst,
the "elementary reaction process collectivity" being a plurality of continuous elementary reaction processes or a collectivity of elementary reaction processes, the elementary reaction process" being what expresses an advancing route of the elementary reaction, the "elementary reaction" being a reaction that the change of a bond is expressed by one microscopic process in a system including at least one chemical species including a molecule, an atom, a solid substance, a radical, and an ion, the "bond" being defined to include a covalent bond, an ionic bond and all other bonds for bonding between chemical species including a molecule, an atom, a solid substance, a radical, and an ion, by action of a intermolecular force or another force,
wherein the system executes:
the step of analyzing each expressible elementary reaction process collectivity by applying a suitable one selected from the experimental chemistry method, the computational chemistry method and the computational physics method to respective elementary reaction processes of each elementary reaction process collectivity, where the "experimental chemistry method" is an analyzing method based on an experiment such as a chemical experiment and a chemical analysis, accompanied with a chemical change, a physical property evaluation using an instrumental analysis, and the result thereof, the "computational chemistry method" is an analyzing method mainly for computing an interaction between chemical species (molecule, atom, solid substance, radical, ion, etc.) on the basis of the quantum mechanics, to simulate an interaction and a reaction between chemical species, the "computational physics method" is an analyzing method mainly for computing an interaction between an electron and a nucleus on the basis of the quantum mechanics, to simulate the physical property having a directly engaging electronic structure, and the "analysis" is to know the change, in the elementary reaction process, of the physical property of respective chemical species (molecule, atom, solid substance, radical, ion, etc.) engaging in the reaction, and the energy state of the system, at a starting point and at an ending point of the elementary reaction process, in a reactive intermediate, and in a transition state,
the step of regarding a combination of the result of the analysis of the respective elementary reaction processes of each elementary reaction process collectivity as the result of the analysis of the elementary reaction process collectivity;
the step of comparing the result of the analysis of each elementary reaction process collectivity with the result of the analysis by the experimental chemistry method for the overall catalytic reaction;
the step of selecting an elementary reaction process collectivity having the highest degree of coincidence, as the most doubtless elementary reaction process collectivity, to reflect the result of the analysis to a selection of controllable parameters of the catalyst, where the "controllable parameter" is a parameter that can be selected within any or a specific range including a reaction condition such as a treating temperature and a treating flow rate, a catalyst construction (including a catalyst composition), and a carrier, in an aimed catalytic reaction process.

14. A system claimed in Claim 13, wherein the system executes:
the step of regarding a combination of the result of the analysis of the respective elementary reaction processes of each elementary reaction process collectivity as the result of the analysis obtained when the respective analyzing methods are assigned to the respective elementary reaction process collectivity;
the step of comparing the result of the analysis of respective elementary reaction process collectivity when the respective analyzing methods are assigned, with the result of the analysis by the experimental chemistry method for the overall catalytic reaction;
the step of selecting an analyzing method assignment giving the result of the analysis having the highest degree of coincidence, as a analyzing method assignment which is estimated to have the highest efficiency;
the step of selecting respective analyzing methods assigned to respective elementary reaction processes in the analyzing method assignment giving the result of the analysis having the highest degree of coincidence, as the analyzing method which is estimated to be the most efficient, to reflect the result of the analysis to a selection of controllable parameters of the catalyst.

15. A system claimed in Claim 13, wherein the system executes:
the step of regarding a combination of the result of the analysis of the respective elementary reaction processes of each elementary reaction process collectivity as the result of the analysis obtained when the respective analyzing methods are assigned to the respective elementary reaction process collectivity;
the step of comparing the result of the analysis of respective elementary reaction process collectivity when the respective analyzing methods are assigned, with the result of the analysis by the experimental chemistry method for the overall catalytic reaction;
the step of selecting an elementary reaction process collectivity having the highest degree of coincidence as the most doubtless elementary reaction process collectivity.
the step of selecting a respective analyzing method assigned to respective elementary reaction process of the elementary reaction process collectivity having the highest degree of coincidence, as the analyzing method which is estimated to be the most efficient for the same elementary reaction process,
the step of regarding the result of the analysis as the result of the analysis of the catalytic reaction, to reflect the result of the analysis to a selection of controllable parameters of the catalyst.

16. A computer program product used in a system for developing a catalyst, the computer program product including a recording medium which can be used in a computer, where a catalytic reaction process being expressed as a collectivity of elementary reaction processes,
the "catalyst" being defined to be a substance that changes the velocity of a chemical reaction by adding the substance into a system in which the chemical reaction can advance thermodynamically, the "catalytic reaction process" being what expresses an advancing route of a catalytic reaction, the "catalytic reaction" being defined to be a chemical reaction that can advance in a system including the catalyst, by action of the catalyst,
the "elementary reaction process collectivity" being a plurality of continuous elementary reaction processes or a collectivity of elementary reaction processes, the elementary reaction process" being what expresses an advancing route of the elementary reaction, the "elementary reaction" being a reaction that the change of a bond is expressed by one microscopic process in a system including at least one chemical species including a molecule, an atom, a solid substance, a radical, and an ion, the "bond" being defined to include a covalent bond, an ionic bond and all other bonds for bonding between chemical species including a molecule, an atom, a solid substance, a radical, and an ion, by action of a intermolecular force or another force,
wherein the system executes:
the step of analyzing each expressible elementary reaction process collectivity by applying a suitable one selected from the experimental chemistry method, the computational chemistry method and the computational physics method to respective elementary reaction processes of each elementary reaction process collectivity, where the "experimental chemistry method" is an analyzing method based on an experiment such as a chemical experiment and a chemical analysis, accompanied with a chemical change, a physical property evaluation using an instrumental analysis, and the result thereof, the "computational chemistry method" is an analyzing method mainly for computing an interaction between chemical species (molecule, atom, solid substance, radical, ion, etc.) on the basis of the quantum mechanics, to simulate an interaction and a reaction between chemical species, the "computational physics method" is an analyzing method mainly for computing an interaction between an electron and a nucleus on the basis of the quantum mechanics, to simulate the physical property having a directly engaging electronic structure, and the "analysis" is to know the change, in the elementary reaction process, of the physical property of respective chemical species (molecule, atom, solid substance, radical, ion, etc.) engaging in the reaction, and the energy state of the system, at a starting point and at an ending point of the elementary reaction process, in a reactive intermediate, and in a transition state,
the step of regarding a combination of the result of the analysis of the respective elementary reaction processes of each elementary reaction process collectivity as the result of the analysis of the elementary reaction process collectivity;
the step of comparing the result of the analysis of each elementary reaction process collectivity with the result of the analysis by the experimental chemistry method for the overall catalytic reaction;
the step of selecting an elementary reaction process collectivity having the highest degree of coincidence, as the most doubtless elementary reaction process collectivity, to reflect the result of the analysis to a selection of controllable parameters of the catalyst, where the "controllable parameter" is a parameter that can be selected within any or a specific range including a reaction condition such as a treating temperature and a treating flow rate, a catalyst construction (including a catalyst composition), and a carrier, in an aimed catalytic reaction process.

17. A computer program product claimed in Claim 16, wherein the system executes:
the step of regarding a combination of the result of the analysis of the respective elementary reaction processes of each elementary reaction process collectivity as the result of the analysis obtained when the respective analyzing methods are assigned to the respective elementary reaction process collectivity;
the step of comparing the result of the analysis of respective elementary reaction process collectivity when the respective analyzing methods are assigned, with the result of the analysis by the experimental chemistry method for the overall catalytic reaction;
the step of selecting an analyzing method assignment giving the result of the analysis having the highest degree of coincidence, as a analyzing method assignment which is estimated to have the highest efficiency;
the step of selecting respective analyzing methods assigned to respective elementary reaction processes in the analyzing method assignment giving the result of the analysis having the highest degree of coincidence, as the analyzing method which is estimated to be the most efficient, to reflect the result of the analysis to a selection of controllable parameters of the catalyst.

18. A system claimed in Claim 16, wherein the system executes:
the step of regarding a combination of the result of the analysis of the respective elementary reaction processes of each elementary reaction process collectivity as the result of the analysis obtained when the respective analyzing methods are assigned to the respective elementary reaction process collectivity;
the step of comparing the result of the analysis of respective elementary reaction process collectivity when the respective analyzing methods are assigned, with the result of the analysis by the experimental chemistry method for the overall catalytic reaction;
the step of selecting an elementary reaction process collectivity having the highest degree of coincidence as the most doubtless elementary reaction process collectivity.
the step of selecting a respective analyzing method assigned to respective elementary reaction process of the elementary reaction process collectivity having the highest degree of coincidence, as the analyzing method which is estimated to be the most efficient for the same elementary reaction process,
the step of regarding the result of the analysis as the result of the analysis of the catalytic reaction, to reflect the result of the analysis to a selection of controllable parameters of the catalyst.
